(19) Europäisches Patentamt / European Patent Office / Office européen des brevets

(11) **EP 3 193 821 B1**

(12) **FASCICULE DE BREVET EUROPÉEN**

(45) Date de publication et mention
de la délivrance du brevet:
**02.01.2019  Bulletin 2019/01**

(21) Numéro de dépôt: **15771178.9**

(22) Date de dépôt: **28.08.2015**

(51) Int Cl.:
*A61K 38/16* *(2006.01)*  *A61K 8/34* *(2006.01)*
*A61K 8/64* *(2006.01)*  *A61Q 19/08* *(2006.01)*
*C07K 14/195* *(2006.01)*  *C07K 14/405* *(2006.01)*
*A61Q 17/04* *(2006.01)*  *A61Q 19/02* *(2006.01)*
*A61K 35/748* *(2015.01)*  *A23L 33/195* *(2016.01)*
*A61K 8/9706* *(2017.01)*  *A61Q 19/00* *(2006.01)*

(86) Numéro de dépôt international:
**PCT/FR2015/052293**

(87) Numéro de publication internationale:
**WO 2016/030643 (03.03.2016 Gazette 2016/09)**

(54) **PROCÉDÉ D'OBTENTION D'UN PRÉCIPITÉ STABLE ENRICHI EN PHYCOBILIPROTÉINES**

VERFAHREN ZUR HERSTELLUNG EINES STABILEN, PHYCOBILIPROTEINANGEREICHERTEN PRÄZIPITATS

METHOD FOR PRODUCING A STABLE PRECIPITATE ENRICHED IN PHYCOBILIPROTEINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **28.08.2014  FR 1401918**

(43) Date de publication de la demande:
**26.07.2017  Bulletin 2017/30**

(73) Titulaire: **Algobiotech**
**91030 Evry Cedex (FR)**

(72) Inventeurs:
• **BEN OUADA, Hatem**
**91030 Evry Cedex (FR)**
• **AMMAR, Jihene**
**91030 Evry Cedex (FR)**

(74) Mandataire: **Marro, Nicolas et al**
**Cabinet Beau de Lomenie**
**158 rue de l'Université**
**75340 Paris Cedex 07 (FR)**

(56) Documents cités:
**WO-A1-2014/045177     CN-A- 1 438 240**
**FR-A1- 2 453 199**

**Description**

La présente invention concerne un procédé d'obtention d'un précipité stable enrichi en phycobiliprotéines, à partir d'algues.

Plus particulièrement, la présente invention concerne un procédé d'obtention d'un précipité stable et enrichi en phycobiliprotéines, comprenant au moins une étape de précipitation consistant en l'ajout d'acide salicylique à un extrait aqueux de phycobiliprotéines, ledit précipité ayant une teneur en phycobiliprotéines au moins égale à 50% de la quantité initialement contenue dans ledit extrait aqueux, et présentant une clarté au moins égale à 70%. Les phycobiliprotéines, dont on en distingue les phycocyanines, les phycoérythrines, les allophycocyanines et leurs protéines associées, sont des protéines pigmentaires photosynthétiques présentes chez certaines algues (algues rouges et cryptophycées) et chez toutes les cyanobactéries. Ces pigments protéiques ont une capacité colorante allant du rouge au bleu.

Actuellement, ces pigments connaissent un regain d'intérêt. Il existe en effet à l'heure actuelle une forte tendance de substituer les composants synthétiques par des produits naturels. Cette tendance s'explique d'une part, par l'augmentation de la demande du consommateur pour des produits naturels pour des impératifs sanitaires, écologiques et de protection de l'environnement et d'autre part, par l'évolution de la législation qui vise à favoriser les produits naturels par rapport à ceux de synthèse. Cet état de fait explique l'accroissement considérable de la demande du marché en colorant naturels.

La revue de la liste des colorants naturels disponibles sur le marché fait apparaître un manque considérable de colorants bleus. Les seuls indiqués sont la Chamazulène (extraite des fleurs de la camomille) et l'Indigo (issu de la fermentation des feuilles de l'indigotier). La disponibilité de ces deux colorants reste cependant en deçà des besoins du marché, en raison des faibles rendements d'extraction qui ne dépassent pas pour les deux cas 0,1% du poids sec.

Parmi les phycobiliprotéines, la phycocyanine détient un monopole de fait en raison de sa couleur bleue unique. La phycocyanine est une protéine colorée trouvée exclusivement chez les algues bleues. Elle n'est pas synthétisable par voie chimique. Les résultats de diverses études portant sur la phycocyanine font état de l'éventualité de son pouvoir antioxydant, anti-inflammatoire et anti-tumoral.

La phycocyanine est largement indiquée pour colorer en bleu certains produits alimentaires (glacés ou sucrés du genre boissons glacés, crèmes glacées, pâtes, gâteaux et biscuits, etc...).

Elle peut être introduite comme composant dans des crèmes de soin de la peau, des masques de beauté et des produits solaires. Elle remplace avantageusement des pigments de synthèse considérés comme suspects.

A l'heure actuelle, les applications connues de la phycocyanine à échelle industrielle se limitent à quelques produits alimentaires (les massepains ; les jus sucrés ; les glaces, sorbets et accessoirement des chocolats) ou à quelques formulations cosmétiques (crèmes et gels). Les seules ventes de la phycocyanine sur le marché sont monopolisés par quelques compagnies (essentiellement japonaises) et n'arrivent à couvrir que 2% au maximum du marché américain de colorants naturels alimentaires.

De plus, la phycocyanine peut être extraite à partir de la cyanobactérie spiruline (*Arthrospira platensis*), pouvant contenir de 3 à 12% de phytocyanine. Cette microalgue bleue considérée par l'OMS comme sans danger (SAFE), est produite à échelle industrielle. Son exploitation est largement répandue dans plusieurs pays du monde.

Une fois les phycobiliprotéines extraites de la biomasse, il est nécessaire de procéder à une étape de concentration qui se traduit le plus souvent par une précipitation.

Il existe plusieurs procédés de concentration des phycobiliprotéines. Certains sont longs utilisant des équipements lourds tel que la centrifugation, la lyophilisation ou encore la dialyse. L'extrapolation de tels procédés à échelle industrielle génère des couts d'investissement élevés. D'autres, plus simples, utilisent la précipitation par les sels et particulièrement le sulfate d'ammonium qui permet en plus de conserver les phycobiliprotéines contre les bactéries et les champignons. Dans le cadre d'une précipitation au sulfate d'ammonium des quantités élevées de ce sel (supérieure à 60% du poids de l'extrait) sont nécessaires. De plus le sulfate d'ammonium est connu pour ses effets aigus d'irritation de la peau et des yeux et de congestions pulmonaires et difficultés respiratoires en cas d'ingestion ou d'inhalation.

On exclut de ce cadre les autres méthodes utilisées communément pour la précipitation des protéines emploient des acides, solvants organiques, polymères non ioniques ou même des agents floculant qui sont non alimentaires, et causent souvent la dénaturation des phycobiliprotéines.

Les inventeurs ont mis en évidence que l'ajout d'acide salicylique à un extrait aqueux de phycobiliprotéines permettait de précipiter les phycobiliprotéines. L'acide salicylique est un acide naturel, de qualité alimentaire et très communément utilisé comme conservateur alimentaire.

Les exigences principales du marché des phycobiliprotéines, couvrent trois aspects essentiels : la nécessité de répondre aux diverses législations relatives aux produits alimentaires ou cosmétiques, une bonne stabilité de la couleur et un prix compétitif. Le caractère naturel des phycobiliprotéines en général, et le fait que leur extraction soit réalisée en solution aqueuse, en l'absence de tout solvant organique, est un avantage certain au niveau de la législation.

Le problème technique que cherche à résoudre l'invention, est l'obtention d'un précipité riche en phycobiliprotéines tout en étant stable.

Les phycobiliprotéines sont de nature protéique, solubles dans des solvants aqueux, ce qui favorise le développement des bactéries et des champignons et affecte sérieusement leur durée de conservation. Les phycobiliprotéines sont par ailleurs, fortement photosensibles ce qui engendre une mauvaise stabilité de la couleur, limitant une utilisation massive de ces phycobiliprotéines en industrie. En effet Les phycobiliprotéines sont sujets à une photo-dégradation leur faisant rapidement perdre leur capacité colorante même sous faible éclairement. Ce processus photochimique conduit à la dénaturation partielle ou totale de la molécule chromophore. Certains procédés utilisent des piégeurs d'oxygène et de radicaux libres, telle que la bioptérine-glucoside. Les phycobiliprotéines sont conjuguées à ces piégeurs par voie chimique. De tels stabilisateurs ne donnent pas, jusqu'à présent, entière satisfaction du fait qu'il n'existe aucune certitude sur leur non toxicité. Par ailleurs, leur coût est trop élevé pour envisager une utilisation à grande échelle quel que soit l'utilisation finale des phycobiliprotéines.

Enfin, les phycobiliprotéines stabilisées jusqu'à présent ne le sont que sur un temps court (quelques heures), incompatible avec une application cosmétique, nutraceutique ou agroalimentaire de ces pigments.

Le brevet US5643585 propose d'utiliser un matériel dérivé d'algues rouges, sous forme non soluble, plus stable ; ledit matériel étant obtenu par un procédé utilisant un solvant organique Cette forme non soluble limite l'utilisation de ces pigments à des composés en poudre.

Le brevet WO2005065697 décrit un procédé de photo-stabilisation de phycobiliprotéines par l'ajout d'un excipient contenant de l'acide ascorbique dans l'extrait aqueux. Ce procédé ne décrit aucune étape de concentration de l'extrait qui est indispensable pour des préparations exigeant une faible teneur en eau.

Le brevet WO2014045177 décrit un procédé d'extraction et de stabilisation de la phycocyanine par macération directe de la biomasse dans du glycérol ou dans un mélange eau/glycérol suivie d'une filtration. Le temps de macération nécessaire est assez long allant jusqu'à 15 jours. De même le rendement d'extraction est assez faible, ne dépassant pas 1% de la biomasse utilisée.

Les inventeurs ont de façon surprenante mis en évidence que l'ajout d'acide salicylique à un extrait aqueux de phycobiliprotéines permettait de résoudre le problème technique posé.

L'invention a donc pour principal objet un procédé d'obtention d'un précipité stable enrichi en phycobiliprotéines, comprenant au moins une étape de précipitation consistant en l'ajout d'acide salicylique à un extrait aqueux de phycobiliprotéines, ledit précipité ayant une teneur en phycobiliprotéines au moins égale à 50% de la quantité initialement contenue dans ledit extrait aqueux et présentant une clarté au moins égale à 70%.

On entend par phycobiliprotéines, les protéines pigmentaires hydrosolubles de la photosynthèse telles que l'allophycocyanine (APC), la famille des phycocyanines (C-PC et R-PC), la famille des phycoérythrines (R-PE et C-PE) et la phycoérythrocyanine (PEC), isolées de certaines microalgues ou de cyanobactéries.

On entend par extrait aqueux, le produit obtenu par l'étape d'extraction et de dissolution des phycobiliprotéines en utilisant un solvant aqueux comme l'eau, l'eau de mer ou toute solution aqueuse contenant $CaCl_2$, NaCl ou tout autre sel de phosphate ou de potassium.

On entend par précipité, ce qui résulte de l'étape de précipitation, à savoir la quantité de phycobiliprotéines concentrée par précipitation, à partir d'un extrait aqueux. Dans le cadre de l'invention on utilise l'acide salicylique pour précipiter les phycobiliprotéines.

On entend par « stable », un précipité dans lequel les phycobiliprotéines ne sont pas dégradées, en particulier dégradées à la lumière naturelle. La stabilité peut être mesurée par la demi-vie des phycobiliprotéines qui représente le nombre de jours aboutissant à la dégradation de la moitié des phycobiliprotéines.

La demi-vie de phycobiliprotéines précipitées par le procédé de la présente invention peut atteindre 50, 60, 70, 80, 90, 100 voire 110 jours pour une température inférieure à 20°C, en particulier une température comprise entre 10 et 20°C et une intensité lumineuse inférieure à 50 $\mu$mol $m^{-2}$ $s^{-1}$, en particulier une intensité lumineuse comprise entre 5 $\mu$mol $m^{-2}$ $s^{-1}$ et 50 $\mu$mol $m^{-2}$ $s^{-1}$.

La demi-vie est par exemple déterminée en suivant la concentration en phycobiliprotéines en fonction des jours (ou des heures). Le taux de dégradation de phycobiliprotéines est alors exprimé ainsi : ((quantité phycobiliprotéines initiale - quantité phycobiliprotéines au jour j )*100 / quantité phycobiliprotéines initiale). La demi vie correspond au nombre de jours ou la moitié (50%) de la concentration initiale est dégradée c'est-à-dire que ce taux est égal à 50%. Dans le cas où la dégradation est faible et n'atteint pas les 50% on peut extrapoler en utilisant le taux de dégradation journalier obtenu dans la gamme expérimentale pour prédire la demi vie en dehors de l'intervalle.

La stabilité peut également être évaluée par le maintien de la couleur. En effet, toute décomposition des phycobiliprotéines entrainerait un changement de couleur de la suspension ou de l'extrait.

La couleur peut être évaluée en mesurant l'absorbance de l'extrait à 620 nm par exemple, correspondant au pic d'absorption des phycobiliprotéines. La diminution de l'absorbance à cette longueur d'onde indique une dégradation des phycobiliprotéines.

Une biomasse de microalgues contient au maximum, selon les conditions de culture, 20 à 25% de phycobiliprotéines. En conséquence, on entend par « précipité ayant une teneur en phycobiliprotéines au moins égale à 50% de la quantité initialement contenue dans l'extrait aqueux », le fait que 50% des 20 à 25% que compte l'extrait aqueux en phycobiliprotéines sont précipités par le procédé selon l'invention.

Les phycobiliprotéines objet de la présente invention peuvent être extraites à partir de cyanobactéries ou de microalgues.

Lesdites microalgues sont en particulier choisies parmi les rhodophycées, les cryptophycées et les cyanobactéries, plus particulièrement les cyanobactéries, encore plus particulièrement *Arthospira Platensis* et *Aphanizomenonflos-aquae,* notamment l'algue Klamath.

Lorsque l'extraction est faite sur *Arthrospira platensis* (spiruline), elle est réalisée selon le procédé décrit par Rebeller M. et col. 1979. Ce procédé d'extraction est réalisé dans une solution de $CaCl_2$ à 10 g/l pendant deux heures, qui est suivie d'une filtration sur toile de 10 $\mu$m. Elle peut être réalisée également dans de l'eau de mer ce qui permet d'enrichir l'extrait phycobiliprotéines en oligoéléments et de réduire le cout d'extraction en évitant l'utilisation des sels de commerce.

La quantité des différents phycobiliprotéines en solution est déterminée par la méthode spectrophotométrique établie par Bryant et col. 1976.

Le procédé selon la présente invention permet d'obtenir un précipité de phycobiliprotéines comprenant au moins 50% de la quantité de phycobiliprotéines contenues dans l'extrait aqueux initial, de préférence, 75% et de manière encore plus préférée 80%.

Dans un mode de réalisation particulier de l'invention, ledit précipité de phycobiliprotéines comprend au moins 85% de la quantité de phycobiliprotéines contenues dans l'extrait aqueux initial, ou au moins 90%, 95% et jusqu'à ce que la précipitation soit totale, c'est-à-dire que ledit précipité comprend 100% des phycobiliprotéines contenues dans l'extrait aqueux. L'extraction des phycobiliprotéines à partir d'une biomasse sèche ou fraiche entraîne une lyse cellulaire.

On entend par lyse cellulaire la rupture des membranes plasmiques de cellules ou de bactéries par tout moyen et notamment des moyens physiques/mécaniques (par sonication, cycles congélation/décongélation) ou des moyens chimiques (solutions de $CaCl_2$, $(NH_4)_2SO_4$) ou encore des moyens biologiques (enzymes). La lyse cellulaire engendre des fragments et des complexes membranaires non solubles, qui subsistent en suspension dans l'extrait récupéré. L'élimination de ces résidus d'extraction est appelée clarification.

Les inventeurs ont de façon surprenante mis en évidence que l'ajout d'acide salicylique à un extrait aqueux de phycobiliprotéines permet à la fois de précipiter les phycobiliprotéines contenues dans l'extrait et d'éliminer les résidus d'extraction.

On peut parler de précipitation sélective séparant les phycobiliprotéines des résidus d'extraction. Il est en effet possible de précipiter l'un ou l'autre de ces deux groupes de composés en variant la concentration en acide salicylique.

On entend par clarté la proportion de phycobiliprotéines dans le précipité total obtenu selon le procédé de l'invention. La clarté est de 100% lorsque le précipité ne contient pas de fragments membranaires résidus de la lyse cellulaire. Après ajout de l'acide salicylique la quantité de phycobiliprotéines et de fragments non précipitées sont déterminées séparément par spectrophotométrie. On en déduit les teneurs en phycobiliprotéines et en fragments précipitées. Et la clarté est mesurée de la manière suivante : 100 * quantité de phycobiliprotéines précipitées/(quantité de phycobiliprotéines +fragments précipités).

La teneur en phycobiliprotéines est estimée en se basant sur la densité optique à 620nm et 650 nm et par l'application des formules de Bryant et col (1976).La teneur en fragments est évaluée par la mesure de la densité optique à 680nm et selon une courbe de corrélation établie liant la $DO_{680}$ à la biomasse sèche de microalgues (Costa et al., 2002).

Le procédé selon la présente invention permet d'obtenir un précipité ayant une teneur en phycobiliprotéines au moins égale à 50% de la quantité initialement contenue dans ledit extrait aqueux et présentant une clarté au moins égale à 80%, de préférence 90%, ou égale à 100%.

Le procédé selon la présente invention, permet d'obtenir un précipité de phycobiliprotéines comprenant de 50% à 80% de la quantité de phycobiliprotéines contenue dans ledit extrait aqueux et présente une clarté au moins égale à 70%.

Dans un mode particulier de réalisation de l'invention, la clarté dudit précipité enrichi en phycobiliprotéines est égale à 75%, 80%, 85%, 90% 95% ou encore 100%. En d'autres termes, la teneur en fragments ou complexes membranaires est comprise entre 0% (la clarification est alors totale) et 30% et peut être égale à 5%, 10%, 15% ou 25% de la quantité de précipité.

Dans un mode préféré de réalisation de l'invention, le procédé permet l'obtention d'un précipité ayant une teneur en phycobiliprotéines au moins égale à 70% de la quantité initialement contenue dans ledit extrait aqueux et étant dépourvu de fragments membranaires. On entend par « dépourvu de fragments membranaires », le fait que le précipité présente une clarté de 100%.

Dans un mode particulier de l'invention, le procédé selon la présente invention utilise un extrait aqueux de phycobiliprotéines contenant au moins 1,5g/l desdites phycobiliprotéines.

L'extrait aqueux de phycobiliprotéines utilisée pour la précipitation à l'aide de l'acide salicylique doit donc contenir une quantité égale à supérieure à 1,5g/l de phycobiliprotéines. La présente invention établit l'équation suivante qui permet de déterminer la quantité minimale d'acide salicylique à utiliser pour n'importe quelle quantité de phycobiliprotéines solubles dans l'extrait initialAcide salicylique (g) = 0,007 * Phycobiliprotéines (mg) / 0,52. L'acide salicylique utilisé dans le procédé selon la présente invention est ajouté à l'extrait aqueux contenant au moins 1,5g/l desdites phycobiliprotéines de manière à obtenir une concentration en acide salicylique sensiblement égale ou supérieure à 4g/l et sensiblement égale ou inférieure à 20g/l. Dans un mode de réalisation particulier de l'invention, la quantité d'acide salicylique ajoutée est comprise entre 4g/l et 10g/l, ou comprise entre 10g/l et 15g/l ou encore entre 15g/l et 20g/l.

Dans un mode de réalisation particulier de l'invention, l'acide salicylique est ajouté à l'extrait aqueux de phycobiliprotéines de manière à obtenir une concentration en acide salicylique sensiblement égale ou supérieure à 4 et sensiblement égale ou inférieure à 13g/l. Dans ce mode de réalisation, le précipité de phycobiliprotéines présente une teneur en phycobiliprotéines au moins égale à 50% de la quantité initiale contenue dans l'extrait aqueux et une clarté de 100%. En d'autres termes, le précipité ne présente plus de fragments membranaires ou résidus issus de l'étape d'extraction permettant l'obtention de l'extrait aqueux.

La clarification est alors totale. Les fragments et complexes membranaires restent alors dans le surnageant.

Plus particulièrement, il a été démontré que la qualité de la clarification (clarification totale ou partielle) est dépendante des teneurs respectives en phycobiliprotéines et en fragments membranaires. En effet, plus la solution est concentrée en phycobiliprotéines et moins elle contient de fragments, et meilleure sera la clarification. Plus précisément, la solution en phycobiliprotéines présentant une teneur en phycobiliprotéines supérieure à 1,5 g/l, de préférence une teneur en phycobiliprotéines allant de 1,5g/l à 1,8g/1, et une teneur en fragments membranaires allant de 0,7g/l à 0,9g/l permettra une bonne clarification. Dans ces conditions la clarification peut être totale pour une concentration en acide salicylique comprise entre une valeur sensiblement égale ou supérieure à 4g/let une valeur sensiblement égale ou inférieur à 13g/l.

Dans un autre mode de réalisation de l'invention, l'acide salicylique est ajouté à l'extrait aqueux de phycobiliprotéines de manière à obtenir une concentration en acide salicylique sensiblement égale ou supérieure à 13g/l et sensiblement égale ou inférieure à 14g/l. Dans ce mode de réalisation, le précipité de phycobiliprotéines présente une teneur en phycobiliprotéines au moins égale à 70% de la quantité initiale contenue dans l'extrait aqueux et une clarté au moins égale à 70%.

Dans un mode particulier de réalisation de l'invention, le procédé peut comprendre une étape de clarification préalable à l'étape de précipitation par l'acide salicylique.

La clarification peut se faire par une simple décantation qui élimine une grande partie des résidus mais dans ces conditions une proportion plus au moins élevée de fragments et de complexes membranaires non solubles, produits de lyse cellulaire, subsistent en suspension dans l'extrait récupéré.

Une clarification totale nécessite le recours à des procédures plus drastiques tell que la centrifugation à plus de 8000t/mn, la dialyse ou l'ultrafiltration tangentielle avec des boyaux ou de membranes qui coupent à moins de 100 KDaltons ou la chromatographie utilisant des polymères qui permettent de retenir les fragments lors du passage de l'extrait. L'extrapolation à échelle industrielle de tels procédés, drastiques, de clarification génère souvent des couts d'investissement élevés.

La clarification peut donc être réalisée par l'ajout d'acide salicylique ou par deux moyens complémentaires, que sont la décantation dans une première étape et l'ajout d'acide salicylique à l'extrait aqueux lors de la précipitation.

Dans un mode particulier de réalisation de l'invention, le procédé peut comprendre une étape de solubilisation du précipité, après la précipitation sélective des phycobiliprotéines à l'aide d'acide salicylique.

Cette étape de solubilisation est notamment réalisée à l'aide d'un polyol naturel, de qualité alimentaire. La glycérine ou le glycérol en sont des exemples. La glycérine est un excellent conservateur naturel. Elle stabilise les protéines et empêche la croissance bactérienne et fongique. La glycérine a l'avantage de permettre une meilleure conservation des phycobiliprotéines.

Le glycérol ou tout dérivé ou toute forme du polyol propan-1,2,3-triol (ou 1,2,3-propanetriol) peuvent aussi être utilisée dans le cadre de l'invention.

Dans le cadre de la présente invention, il a été déterminé que 1,2 1 de glycérine permet de solubiliser 27 g de phycobiliprotéines précipitées et/ou clarifiées.

La solution phycobiliprotéines-glycérine peut être conservée plus de trois mois à des conditions ambiantes de température et luminosité et plusieurs années à froid et à l'obscurité.

Le procédé de la présente invention peut également être mis en oeuvre suite à une étape d'induction de la synthèse des phycobiliprotéines dans une biomasse dont la croissance est bloquée. Le procédé de la présente invention permet d'obtenir une biomasse présentant une teneur en phycobiliprotéines au moins égale à 20% du poids sec de ladite biomasse.

Le procédé de la présente invention permet en particulier d'obtenir une biomasse présentant une teneur en phycobiliprotéines au moins égale à 21%, 22%, 23%, 24% ou 25% du poids sec de ladite biomasse.

Le procédé de la présente invention permet en particulier d'obtenir une biomasse présentant une teneur en phycobiliprotéines comprise de 20% à 25% du poids sec de ladite biomasse. Cette étape du procédé étant réalisé en dehors du bassin de culture, il permet de ne pas altérer la productivité de la biomasse.

On entend par « biomasse », la masse totale des microalgues vivantes à un moment donné dans une culture. Elle est exprimée en gramme par litre (g/l).

On entend par « phycobiliprotéines », les protéines pigmentaires hydrosolubles de la photosynthèse telles que l'allophycocyanine (APC), la famille des phycocyanines (C-PC et R-PC), la famille des phycoérythrines (R-PE et C-PE) et la phycoérythrocyanine (PEC), isolées de certaines microalgues ou de cyanobactéries.

On entend par « induction de la synthèse », la stimulation par tout moyen de la production de phycobiliprotéines par une biomasse de microalgues.

On entend par « milieu d'induction » tout milieu de culture de microalgues contenant un ou plusieurs sels en excès ou en déficit par rapport à la concentration en sels nécessaire à la croissance optimale. Dans le cadre de l'invention ce milieu contient un excès d'azote obtenu par l'ajout de $NaNO_3$ au milieu de culture Zarrouk, à une concentration supérieure à 2.5 g/L et de préférence comprise de 3 et 5 g/L.

On entend par « blocage de croissance », le fait de stopper la multiplication de la microalgue. Le blocage de la croissance est réversible. En effet, suite au blocage de la croissance, une partie de la biomasse est collectée. La concentration est alors ainsi diminuée et la biomasse peut de nouveau croitre. La biomasse n'est donc pas altérée.

L'étape de blocage de la croissance de ladite biomasse de microalgues est réalisée dans un bassin d'induction, et est réalisée lorsque la concentration de ladite biomasse dans ledit bassin est de 3 à 13 fois plus élevé que la concentration permettant la croissance optimale des micro algues en culture.

Le blocage de la croissance de ladite biomasse de microalgues, en particulier de cyanobactéries, plus particulièrement *d'Arthospira Platensis,* est réalisé dans un bassin d'induction, ladite concentration de ladite biomasse dans ledit bassin étant en particulier 3, 4, 5, 6, 7, 8, 9, 10, 11, 12 ou 13 fois plus élevée que la concentration permettant la croissance optimale des microalgues en culture.

La détermination du blocage de croissance (le fait que la multiplication des microalgues soit bloquée) se fait par exemple en suivant la courbe de vitesse de croissance des microalgues en fonction de la densité de culture. Un blocage est observé lorsque la vitesse de croissance des micro algues est nulle.

Le terme « concentration » peut être remplacé par le terme « densité ». On parle indifféremment de « densité de la biomasse » ou de « concentration de la biomasse ». La densité comme la concentration sont exprimées en g/l.

Il est admis que la densité/concentration permettant la croissance optimale des microalgues en culture est d'environ 0,4 g/l.

Ce blocage est réalisé par l'introduction de la biomasse à une concentration allant de 1 à 5 g/l dans un bassin d'induction, mais ce moyen est non limitatif et tout moyen de blocage de la croissance de la biomasse peut être utilisé dans le cadre de l'invention. La concentration de ladite biomasse au moment du blocage peut être égale à 1 g/l, 1,5 g/l, 2 g/l, 2,5 g/l, 3 g/l, 3,5 g/l, 4 g/l, 4,5 g/l ou encore 5 g/l.

La densité/concentration de la biomasse peut être mesurée par différentes méthodes.

- Le disque de Secchi : C'est un dispositif simple permettant d'estimer rapidement la densité des cellules cultivées en milieu aquatique. Il est constitué d'une règle graduée de 30cm de long, munie d'un disque blanc de 5cm de diamètre à l'extrémité inférieure, au point zéro. On note la profondeur, en centimètres, à partir de laquelle on ne peut plus distinguer le disque une fois plongé dans le milieu.

- Les comptages sous microscope. Cette méthode est coûteuse en temps, mais elle permet d'estimer le nombre de filaments et le nombre de spires par filament. A l'aide d'une pipette calibrée, on dépose une goutte d'échantillon sur une lame creuse pour l'observation sous microscope; on évalue le nombre de filaments dans une goutte, sachant que 17 gouttes de notre pipette représentent un volume de 1 ml. Si la culture est très concentrée, on dilue l'échantillon. Le nombre moyen de spires est calculé sur 30 filaments pris au hasard.

- La densité optique à 665 nm. Cette méthode permet d'estimer rapidement la biomasse en utilisant l'absorption à 665 nm, qui est une des longueurs d'onde d'absorption de la chlorophylle. Cette absorption in vivo est généralement bien corrélée à la concentration en chlorophylle. On utilise un spectrophotomètre équipé d'une cuve à faces parallèles de 25 cl. Le zéro est fait sur du milieu de culture non ensemencé.

Le bassin d'induction peut être un bassin de culture intermédiaire ou un bassin de récupération ou tout autre système clos de stockage de microalgues vivantes.

Dans un mode de réalisation préféré de l'invention, la concentration de ladite biomasse dans le bassin d'induction est comprise entre une valeur sensiblement égale ou supérieure à 1g/L et une valeur sensiblement égale ou inférieure à 5 g/L. Cette concentration est obtenue en additionnant du milieu d'induction dans le bassin d'induction.

La concentration allant de 1g/L à 5g/L correspondant à une densité de 3 à 13 fois supérieure à la densité de la biomasse en croissance au moment de la collecte, c'est-à-dire au moment de la croissance optimale où la concentration est d'environ 0,4g/l. A cette concentration, la croissance est bloquée pour favoriser le métabolisme de défense de la microalgue.

La croissance d'une biomasse, par exemple en croissance optimale, notamment à une concentration d'environ 0,4g/l peut être bloquée en portant ladite biomasse à une concentration comprise de 1g/L à 5g/L par exemple par filtration, décantation ou centrifugation.

La biomasse peut également subir un lavage avant son dépôt dans le bassin d'induction. Le lavage permet d'éliminer les sels en excès provenant du milieu de culture. Ceci permet ainsi un meilleur contrôle de la concentration en azote de la solution d'induction.

Dans ce cas la biomasse est lavée 3 fois en utilisant une eau physiologique ou la solution du milieu d'induction fraichement préparée.

Dans ces conditions, la biomasse initiale n'est pas affectée par le procédé et l'induction de la synthèse des phycocyanines n'est pas dépendante des conditions de culture de la biomasse. Cette procédure présente l'avantage de pouvoir être facilement intégrée dans la chaine de production d'une ferme traditionnelle de culture de spiruline. Elle peut être appliquée dans un bassin de culture intermédiaire ou dans un bassin de récupération pour une durée de 3 heures, 4 heures ou jusqu'à 5 heures.

Dans un mode de réalisation particulier de l'invention, l'étape d'induction de la synthèse des phycobiliprotéines comprend :

1) l'exposition de la biomasse de microalgues photo synthétiques à un flux lumineux, ledit flux ayant une intensité lumineuse comprise d'une valeur sensiblement égale ou supérieure à 10 $\mu$mol $m^{-2} s^{-1}$ à une valeur sensiblement égale ou inférieure à 13 $\mu$mol $m^{-2} s^{-1}$

2) l'ajout d'une source d'azote de manière à obtenir une concentration en $NaNO_3$ dans le milieu d'induction supérieure à 2.5 g/L

L'intensité lumineuse en $\mu$moles de photons par mètre carré et par seconde, est par exemple déterminée à l'aide d'un photomètre mesurant les radiations actives pour la photosynthèse.

A noter que 10 $\mu$mol $m^{-2} s^{-1}$ correspondent à 330 Lux et que 13 $\mu$mol $m^{-2} s^{-1}$ correspondent à 429 Lux, en particulier avec pour éclairage un tube fluorescent, par exemple de type « Plant Growth fluorescent ». Dans ce cas, 1 $\mu$mole de photons par mètre carré et par seconde équivaut à 33 lux.

La concentration en NaNO3 dans le milieu d'induction est notamment comprise de 3 à 4g/L ou de 3 à 5g/l.

La combinaison d'une intensité lumineuse comprise dans intervalle allant de 10 à 13 micromoles $m^{-2} s^{-1}$ ($\mu$mol $m^{-2} s^{-1}$) et d'une solution de culture contenant 3 à 5g/L de $NaNO_3$ (comme source d'azote) permet l'obtention d'une biomasse contenant de 20 à 25% de phycocyanine, ce qui est particulièrement élevée par rapport à l'intervalle de 3 à 12% obtenu dans les expériences mises en oeuvre sans application du procédé de l'invention.

La lumière peut être assurée par des lampes à incandescence simple de puissance 40W ou par des tubes fluorescents.

La solution d'induction préférée est constituée par le milieu de culture Zarrouk, mais tout autre milieu de culture adapté à la spiruline peut être utilisé. Dans tous les cas, la concentration en azote de la solution d'induction est modifiée par l'ajout de nitrate de sodium ($NaNO_3$) en excès. La concentration finale doit être comprise dans un intervalle allant de 3 à 5g/l de NaNO₃, ce qui représente une concentration 1.2 à 2 fois supérieure à celle présente dans le milieu Zarouk initial (2.5g/l de $NaNO_3$).

Dans les conditions de culture définies ci-dessus, l'étape d'induction est réalisée pendant une durée de 3 heures, 4 heures ou jusqu'à 5 heures et est suffisante pour induire la synthèse des phicobiliprotéines à une teneur stable comprise entre 20 et 25% de la biomasse sèche.

Le procédé selon l'invention, de préparation d'une biomasse de microalgues photosynthétiques présentant une teneur en phycobiliprotéines au moins égale à 20% du poids sec de ladite biomasse, comprend une étape de blocage de la croissance de la biomasse et une étape d'induction de la synthèse des phycobiliprotéines.

Selon un mode de réalisation avantageux, la biomasse présente une teneur en phycobiliprotéines au moins égale à 21%, 22%, 23%, 24% ou 25% du poids sec de ladite biomasse.

Selon un mode de réalisation avantageux, la biomasse présente une teneur en phycobiliprotéines comprise de 20% à 25% du poids sec de ladite biomasse.

Dans un mode de réalisation particulier, l'étape d'induction de la synthèse des phycobiliprotéine, peut être précédée par une étape de collecte d'une biomasse de microalgues. La biomasse est collectée dans des bassins de cultures usuels, lorsque la culture est en phase exponentielle de croissance, c'est à dire lorsque la productivité en biomasse est maximale. Le volume récupéré est filtré sur toile (ou par décantation ou par centrifugation) pour obtenir la biomasse fraiche. La biomasse obtenue est transférée dans le bassin d'induction.

Le procédé selon l'invention peut également comprendre une étape finale de collecte de la biomasse enrichie.

Le procédé selon l'invention peut donc comprendre les étapes de

- a) collecte d'une biomasse de microalgues

- b) blocage de la croissance de ladite biomasse

- c) induction de la synthèse des phycobiliprotéines

- d) collecte de ladite biomasse enrichie en phycobiliprotéines

Le procédé selon l'invention utilise les microalogues photosynthétiques choisies parmi les cyanobactéries, les rhodophytes ou les cryptophytes.

De manière préférée, la microalgue photosynthétique utilisée pour mettre en oeuvre le procédé selon l'invention est *Arthospira Platensis,* aussi appelée spiruline.

Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

Également décrit est un précipité stable de phycobiliprotéines tel qu'obtenu par le procédé tel que décrit précédemment, sous forme de poudre ou en suspension. Les phycobiliprotéines clarifiées et stabilisées sont en effet utilisables pour toute forme d'application (poudre, liquide ou semi-liquide) tant dans le domaine de la cosmétique, que dans le cadre de préparations nutraceutiques, agroalimentaires, ou pharmaceutiques.

Les phycobiliprotéines précipitées à l'acide salicylique par le procédé de l'invention, qu'elles soient clarifiées ou non préalablement par une étape de décantation, peuvent être séchées à l'étuve à 40°C ou lyophilisées, ce qui améliore la stabilité et augmente la durée de conservation. Elles peuvent être incorporées directement dans tout produit en poudre pour toute application agroalimentaire, thérapeutique, cosmétique ou pharmaceutique.

Le précipité solubilisé et stabilisé par la glycérine selon le procédé de l'invention peut être conditionné directement dans des ampoules par exemple ou incorporé dans toute application, liquide ou visqueuse, comme additif, colorant et principe actif pour des formulations alimentaires, cosmétiques ou pharmaceutiques.

Les deux formes des phycobiliprotéines en poudre ou en solution dans la glycérine contiennent en plus, des protéines associées, des résidus du sel $CaCl_2$ ayant servi à l'extraction initiale et des résidus de l'acide salicylique utilisé pour la précipitation. Ces éléments sont tous des produits alimentaires qui ne gênent en rien l'utilisation du produit dans toute application alimentaire, cosmétique ou pharmaceutique.

Dans une variante, on peut réduire la teneur en sel et en acide salicylique par un lavage du précipité avec de l'eau distillée avant sa solubilisation dans la glycérine. Ce lavage est préférable pour des applications ne tolérant pas la présence excessive d'acide salicylique.

Il est visé également en tant que nouveaux produits la solution surnageant obtenue à l'issue de l'étape de précipitation et/ou de clarification. Cette solution contient la fraction non précipitée de phycobiliprotéines et de fragments. Elle est de plus riche en protéines hydrosolubles, en oligoéléments et en une quantité d'acide salicylique solubilisé. Elle forme une composition riche en principes actifs qui peut servir pour toute forme liquide de produits alimentaire et/ou cosmétique.

Également décrits sont des précipités stables enrichis en phycobiliprotaines, sous forme de poudre ou en suspension et totalement dépourvu de fragments membranaires susceptibles d'être obtenu par le procédé de la présente invention.

Enfin, des compositions cosmétique, ou nutraceutique, contenant ledit précipité stable enrichi en phycobiliprotéines, ou un mélange de précipités stables enrichis en phycobiliprotéines, que ce soit des compositions en forme de poudre, comprimés ou gélules ou encore sou forme liquide ou visqueuse telle que les crèmes, gels ou pâtes sont décrits.

La composition cosmétique possède des propriétés anti-rides intensives permettant de lutter contre les rides profondes, le relâchement cutané et le teint terme.

La composition cosmétique est riche en composés anti-oxydants. Elle agit contre les radicaux livres et stimlent l'oxygénation cellulaire. Avec une application continue, le contour du visage est redessiné, les rides profondes sont réduites et le teint est plus uniforme. La composition cosmétique permet aussi de réduire les poches et cernes sous les yeux et protège de la déshydratation pour un meilleur confort.

La composition cosmétique a également des effets anti-tâches et stimule la détoxification cellulaire, protège des effets du soleil et de la pollution, accroit la capacité de lutte contre les taches caractérisées par un excès de mélanine.

Selon l'intensité des taches, deux à quatre applications quotidiennes sont nécessaires. Les taches claires et diffuses disparaissent en quelques semaines alors que les taches foncées et à contour défini peuvent nécessiter 6 à 12 mois pour disparaitre complètement. La composition cosmétique ou dermatologique est également conseillée pour les taches résiduelles des fortes poussées d'acné. Par ailleurs, elle a un effet hydratant pour une peau souple et douce.

Également décrite est une composition dermatologique contenant à titre de substance active ledit précipité stable enrichi en phycobiliprotéines, tel que défini précédemment, ou un mélange de précipités stables enrichis en phycobiliprotéines, et un véhicule pharmaceutiquement acceptable.

Également décrite est l'utilisation d'un précipité stable enrichi en phycobiliprotéines, tel que défini précédemment, ou un mélange de précipités stables enrichis en phycobiliprotéines, en tant qu'antioxydant.

## DESCRIPTION DES FIGURES

La figure 1 représente la courbe d'iso- demi-vie des phycobiliprotéines solubilisées en fonction de la variation de la température (Temp en °C) et de l'intensité lumineuse (Lum en µmoles photon/m2/s).

La figure 2 représente la variation du taux de précipitation des phycobiliprotéines (phy) et des fragments (frag) en fonction de la concentration en acide salicylique (g/l).

La figure 3 représente la variation de la clarté du précipité en fonction de la concentration de fragments (g/l) et des concentrations initiales en phycobiliprotéines. La teneur en acide salicylique est de 13 g/l.

## EXEMPLES

### Exemple 1 : La précipitation à l'acide salicylique

Le procédé de la présente invention est subdivisé en deux étapes essentielles :

1) L'extrait de phycobiliprotéines est additionné d'acide salicylique en poudre de qualité commerciale. La solution (acide salicylique + extrait) est mise sous agitation pendant 15 minutes. Cette étape permet d'homogénéiser la solution et de favoriser la mise en contact de l'acide avec les molécules de phycobiliprotéines en solution.

La solution est mise dans une ampoule à décanter. La quasi-totalité des phycobiliprotéines, plus de 90%, précipite au bout d'une heure au maximum. La quantité d'acide salicylique nécessaire à la précipitation de la quasi-totalité des phycobiliprotéines au bout d'une heure dépend de la quantité de ces derniers dans l'extrait initial. La présente invention établit l'équation suivante qui permet de déterminer la quantité minimale d'acide salicylique à utiliser pour n'importe qu'elle quantité de phycobiliprotéines solubles dans l'extrait initial.

$$\text{Acide salicylique (g)} = 0{,}007 * \text{Phycobiliprotéines (mg)} / 0{,}52.$$

Pour des utilisations exigeant une plus grande pureté des phycobiliprotéines, l'invention montre que l'extrait de phycobiliprotéines est clarifié lorsqu'on applique la précipitation sélective en utilisant l'acide salicylique. La clarification est utilisée après une étape de décantation permettant d'éliminer les résidus décantables de l'extrait. Elle vise par conséquent à séparer les phycobiliprotéines, des fines particules membranaires qui restent en suspension après décantation.

Les phycobiliprotéines précipitées, clarifiés ou non par décantation, sont récupérés par filtration grossière sur toile ou papier filtre ou simplement en déversant le surnageant par le haut de l'ampoule à décanter.

2) Les phycobiliprotéines précipités, clarifiés ou non par décantation, sont solubilisés dans la glycérine. L'invention détermine que 1.2 l de glycérine sont suffisants pour solubiliser 27 g de phycobiliprotéines précipitées. Le rendement de solubilisation est entre 95 % à 99 %. Il est à signaler que la fraction non solubilisée dans la glycérine peut être récupérée dans de l'alcool absolu.

**Exemple 2** : La demi-vie des phycobiliprotéines précipités par l'acide salycylique et resolubilisées dans le glycérol selon le procédé de la présente invention, est étudiée, en fonction de la température et de l'intensité lumineuse.

On désigne par demi-vie (Y) le nombre de jours aboutissant à la dégradation de la moitié des phycobiliprotéines.

La température est étudiée dans un intervalle de 20 à 60°C, l'intensité lumineuse est étudiée dans un intervalle de 50 à 150 µmol photon/m$^2$/s.

Onze expériences ont été réalisé dont huit représentent la combinaison entres les trois niveaux correspondant aux deux facteurs. Les trois autres représentent des points centraux et permettent d'évaluer l'erreur expérimentale.

Le modèle mathématique proposé est :

$$\text{Log}_{10}\text{Y} = 4 - 0.08\text{Temp} - 0.013*\text{Lum} + 0.0005*\text{Temp}^2 + 0.0002*\text{temp}*\text{Lum}.$$

Ce modèle est valide à 94%, avec un coefficient de corrélation $R^2$ = 98% et il est prédictible (Q) à 95%.

La variation de la demi-vie des phycobiliprotéines (en jours) selon les conditions de température et de lumière est donnée par la courbe d'iso demi-vie (figurel)

La demi-vie des phycobiliprotéines précipités par l'acide salicylique et re-solubilisés dans le glycérol peut dépasser 110 jours, pour une température inférieure à 20°C et une intensité lumineuse inférieure à 50 $\mu$mol photon/m$^2$/s.

Par prédiction en dehors de l'intervalle d'étude on peut estimer la demi-vie des phycobiliprotéines stabilisées, à 11 ans lorsque les conditions de stockage sont de 4 °C et dans l'obscurité totale

Dans les mêmes conditions de température (20°C) et de luminosité (50 $\mu$mol photon/m$^2$/s). les essais de stabilité montrent que pour les extraits aqueux non traités, c'est-à-dire n'ayant pas été soumis au procédé de l'invention basé sur l'ajout d'acide salicylique et/ou de la glycérine, la totalité des phycobiliprotéines est dégradée au bout de 12 heures, Pour les phycobiliprotéines en poudre, précipités et stabilisés par l'acide salicylique sans resolubilisation dans le glycérine, la dégradation ne dépasse pas 10% après plus de 2 mois d'incubation.

Pour les extraits aqueux solubilisés dans de la glycérine mais non précipité par l'acide salicylique selon le procédé de la présente invention, la dégradation est de 50%, après un mois d'incubation.

Les extraits aqueux précipités par l'acide salicylique et solubilisés dans la glycérine montrent une meilleure stabilité.

**Exemple 3**

On cherche à clarifier les phycobiliprotéines extraits par précipitation sélective à l'acide salicylique. L'extraction peut se faire à partir de la biomasse sèche ou fraiche de la spiruline. On utilise 13 g de spiruline sèche (ou 130g de spiruline fraiche) suspendue dans 0.2 l d'eau de mer stérile, bien homogénéiser.

L'ensemble est Congelé à -4 °c pendant une nuit. Il est décongelé dans 0.4 l d'eau de mer stérile et soumis à une agitation pendant deux heures à froid. Une deuxième congélation-décongélation suivie d'une décongélation dans 0.4 l d'eau de mer stérile sous agitation et à froid pendant deux heures est effectuée.

La suspension obtenue est transvasée dans un récipient à décanter pendant une nuit à froid afin de séparer la phase aqueuse de l'extrait, de la biomasse décantable.

Le rendement de l'extraction des phycobiliprotéines est estimé à 12 - 15 % du poids sec de la spiruline.

La teneur en phycobiliprotéines est estimée en se basant sur la densité optique à 620nm et 650nm et par l'application des formules de Bryant et col (1976). La teneur en fragments est évaluée par la mesure de la densité optique à 680nm selon une courbe de corrélation établie liant la DO680 à la biomasse sèche de spirulines.

La concentration en phycobiliprotéines de l'extrait, ainsi évaluée, est de 1.5 g/l d'eau de mer. La teneur en fragments et en éléments membranaires est évaluée à 1.9 g/l.

On ajoute par intermittence 1 à 2 g d'acide salicylique en poudre sous agitation continue.

A chaque ajout, la solution est mise au repos pendant deux heures, au bout desquelles les phycobiliprotéines précipitent. On évalue alors la quantité de phycobiliprotéines et de fragments en solution pour déterminer les taux de précipitation.

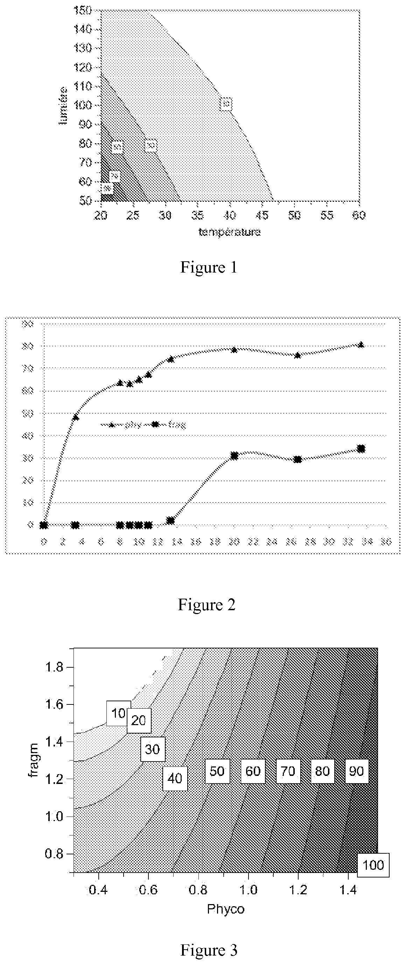

Les résultats des taux de précipitation sont présentés sur la figure 2.

On montre qu'on peut obtenir un précipité à 100% clarifié pour une concentration inférieure à 12-13 g/l d'acide salicylique.

Le rendement maximal de phycobiliprotéines précipitées et totalement clarifiées, ainsi obtenu est de 70% pour un ajout de 13g/l d'acide salicylique.

La totalité des fragments et des complexes membranaires restent dans le surnageant et ne sont précipités qu'à des concentrations élevés en acide salicylique.

**Exemple 4 : Variation du taux de clarté des phycobiliprotéines précipités**

On étudie la variation du taux de clarté des phycobiliprotéines précipitées pour différentes concentrations initiales de l'extrait en phycobiliprotéines et en fragments (obtenus par différents dilution et centrifugation) et pour différentes concentrations d'acide salicylique. L'étude est réalisée selon la méthodologie des plans d'expérience, moyennant un plan factoriel complet à trois niveaux avec interaction. Les facteurs sont : la concentration initiale de l'extrait en phycobiliprotéines, variant de 0.3 à 1.52 g/l, la concentration en fragments variant de 0.7 à 1.9 g/l et la concentration en acide salicylique dans un intervalle de 10 à 20 g/l.

La matrice expérimentale donnée par le modèle est composée de quinze expériences représentent la combinaison entres les trois niveaux (minimum, maximum et centre) correspondant aux trois facteurs. Trois répétitions de la même combinaison permettent d'évaluer l'erreur expérimentale.

Après ajout de l'acide salicylique la quantité de phycobiliprotéines et de fragments non précipitées sont déterminées séparément pour chaque expérience par spectrophotométrie. On en déduit les teneurs en phycobiliprotéines et en fragments précipitées. On calcule la clarté. On désigne par clarté = 100 * quantité de phycobiliprotéines précipitées/quantité de phycobiliprotéines +fragments précipités

Le modèle permet de tracer les courbes d'iso-clarté qui sont présentées dans la figure 3.

On montre qu'on ne peut obtenir un taux de clarification totale de 100% que pour une concentration en phycobiliprotéines supérieure à 1.5 g/l et une teneur en fragments inférieure à 0.9 g/l. La teneur en acide salicylique nécessaire à la clarification totale dans ces conditions est inférieure à 13g/l.

**Exemple 5 : Crème anti rides**

Une crème anti rides est obtenue avec la composition suivante :

Aqua: 59%,
Huile d'amande douce: 18%,
Précipité stable enrichi en phycobiliprotéines solubilisé dans la glycérine : 7%,
Huile de palme: 5%, Huile de ricin: 3%,
Extrait d'algues: 3% (beta carotène),
Cire d'abeille: 2%,
gomme xanrthane: 1,7%,
Parfum: 1%,
éthyl parabène: 0,1%, méthyl parabène: 0,1%, benzyl parabène : 0,1%.

**Exemple 6 : Crème anti taches**

Une crème anti taches est obtenue avec la composition suivante :
Crème anti taches:

Aqua: 59%,
Huile d'amande douce: 19,5%,
Précipité stable enrichi en phycobiliprotéines solubilisé dans la glycérine : 7%,
Huile de palme: 5%, Huile de ricin: 3%,
Extrait d'algues: 1,5% (beta carotène),
Cire d'abeille: 2%,
gomme xanrthane: 1,7%,
Parfum: 1%,
éthyl parabène: 0,1%, méthyl parabène: 0,1%, benzyl parabène : 0,1%.

## REFERENCES

Arad et Ai., 1997. Coloring material. US patent. US/ 5.643. 585

Bryant D.A., Glazer, A.N. et Eiserling F.A., 1976. Characterisation and Structural properties of the major biliproteins of Anabaena sp. Arch. Microbiol, 110,61-75.

Costa JAV, Colla LM, Duarte Filho PF, Kabke K, Weber A., 2002. Modelling of Spirulina platensis growth in freshwater using response surface methodology. World J Microbiol Biotechnol 18:603-607

Minard. F., 2005. Method for the photostabilisation of phycobiliproteins in an aqueous extract, compositions containing stabilised phycobiliproteins and use of stabilised phycobiliproteins. Brevet n° WO2005065697 A1.

Rebeller M. Yout P et Lonchamp D., 1979. Procédé d'extraction selective des colorants contenus dans les algues cyanophycées. (Brevet déposé par l'institut français du pétrole inscrit sous la classification. C 09 B 61/00).

Frédéric Pottecher, 2014. Procédé d'extraction et de stabilisation dephycocyanine et ses application. WO 2014045177 A1. Brevet déposé par Ecosystem.

## Revendications

1. Procédé d'obtention d'un précipité stable et enrichi en phycobiliprotéines, comprenant au moins une étape de précipitation consistant en l'ajout d'acide salicylique à un extrait aqueux de phycobiliprotéines, ledit précipité ayant une teneur en phycobiliprotéines au moins égale à 50% de la quantité initialement contenue dans ledit extrait aqueux,

et présentant une clarté au moins égale à 70%.

2.  Procédé selon la revendication 1 dans lequel ledit précipité a une teneur en phycobiliprotéines au moins égale à 50% de la quantité initialement contenue dans ledit extrait aqueux et présente une clarté au moins égale à 80%, de préférence 90%, ou égale à 100%.

3.  Procédé selon la revendication 1 dans lequel ledit précipité a une teneur en phycobiliprotéines au moins égale à 70% de la quantité initialement contenue dans ledit extrait aqueux et est dépourvu de fragments membranaires.

4.  Procédé selon l'une des revendications précédentes, dans lequel ledit extrait aqueux de phycobiliprotéines contient au moins 1.5g/l desdites phycobiliprotéines.

5.  Procédé selon la revendication 1 dans lequel l'acide salicylique est ajouté à l'extrait aqueux de phycobiliprotéines, dans un intervalle allant de 4 g/l à 20g/l.

6.  Procédé selon la revendication 1 ou 3, dans lequel l'acide salicylique est ajouté à l'extrait aqueux de phycobiliprotéines dans un intervalle allant de 4 g/l à 13g/l.

7.  Procédé selon la revendication 1 ou 2 dans lequel l'acide salicylique est ajouté à un extrait aqueux de phycobiliprotéines dans un intervalle allant de 13 à 14g/l.

8.  Procédé selon l'une des revendications précédentes, dans lequel une étape de clarification préalable par décantation à l'étape de précipitation par l'acide salicylique, est ajoutée.

9.  Procédé selon l'une des revendications précédentes, dans lequel une étape de solubilisation du précipité est ajoutée.

10. Procédé selon la revendication 9, dans lequel le précipité est solubilisé dans un polyol naturel choisi parmi la glycérine, le glycérol, ou tout dérivé ou toute forme du polyol propan-1,2,3-triol (ou 1,2,3-propanetriol).

11. Procédé selon la revendication 1, qui comprend au préalable une étape d'enrichissement en phycobiliprotéines d'une biomasse de microalogues photosynthétiques.

12. Procédé selon la revendication 11, dans lequel l'étape préalable d'enrichissement en phycobiliprotéines comprend l'une induction de la synthèse des phycobiliprotéines dans une biomasse dont la croissance est bloquée.

13. Procédé selon l'une des revendications 1, 11 ou 12 qui comprend :

    a. une étape blocage de la croissance de la biomasse
    b. une étape d'induction de la synthèse des phycobiliprotéines
    c. une étape de précipitation à l'aide d'acide salicylique
    d. une étape de solubilisation du précipité obtenu à l'étape c.

14. Procédé selon l'une des revendications précédentes, dans lequel l'extrait aqueux de phycobiliprotéines est obtenu à partir de microalgues photosynthétiques *Arthospira Platensis.*

**Patentansprüche**

1.  Verfahren zum Erhalt eines stabilen und mit Phycobiliproteinen angereicherten Präzipitats, umfassend zumindest einen Schritt der Präzipitation, der aus der Zugabe von Salicylsäure zu einem wässrigen Extrakt von Phycobiliproteinen besteht, wobei das Präzipitat einen Gehalt an Phycobiliproteinen von zumindest gleich 50 % der ursprünglich in dem wässrigen Extrakt enthaltenen Menge aufweist, und eine Klarheit von zumindest gleich 70 % zeigt.

2.  Verfahren nach Anspruch 1, wobei das Präzipitat einen Gehalt an Phycobiliproteinen von zumindest gleich 50 % der ursprünglich in dem wässrigen Extrakt enthaltenen Menge aufweist, und eine Klarheit von zumindest gleich 80 %, vorzugsweise 90 %, oder gleich 100 % zeigt.

3.  Verfahren nach Anspruch 1, wobei das Präzipitat einen Gehalt an Phycobiliproteinen von zumindest gleich 70 % der ursprünglich in dem wässrigen Extrakt enthaltenen Menge aufweist und frei von Membranfragmenten ist.

4. Verfahren nach einem der vorhergehenden Ansprüche, wobei das wässrige Extrakt von Phycobiliproteinen zumindest 1,5 g/l der Phycobiliproteine enthält.

5. Verfahren nach Anspruch 1, wobei die Salicylsäure zu dem wässrigen Extrakt von Phycobiliproteinen in einem Intervall zugegeben wird, das von 4 g/l bis 20 g/l reicht.

6. Verfahren nach Anspruch 1 oder 3, wobei die Salicylsäure zu dem wässrigen Extrakt von Phycobiliproteinen in einem Intervall zugegeben wird, das von 4 g/l bis 13 g/l reicht.

7. Verfahren nach Anspruch 1 oder 2, wobei die Salicylsäure zu einem wässrigen Extrakt von Phycobiliproteinen in einem Intervall zugegeben wird, das von 13 g/l bis 14 g/l reicht.

8. Verfahren nach einem der vorhergehenden Ansprüche, wobei ein vorausgehender Schritt der Klärung durch Dekantieren zu dem Schritt der Präzipitation durch Salicylsäure hinzugefügt wird.

9. Verfahren nach einem der vorhergehenden Ansprüche, wobei ein Schritt der Solubilisierung des Präzipitats hinzugefügt wird.

10. Verfahren nach Anspruch 9, wobei das Präzipitat in einem natürlichen Polyol solubilisiert wird, das ausgewählt ist aus Glyzerin, Glycerol oder beliebigen Derivaten oder beliebigen Formen des Polyols Propan-1,2,3-triol (oder 1,2,3-Propantriol).

11. Verfahren nach Anspruch 1, das vorab einen Schritt der Anreicherung von Phycobiliproteinen einer Biomasse aus photosynthetisierenden Mikroalgen umfasst.

12. Verfahren nach Anspruch 11, wobei der vorausgehende Schritt der Anreicherung von Phycobiliproteinen eine Induzierung der Synthese der Phycobiliproteine in einer Biomasse umfasst, deren Wachstum blockiert ist.

13. Verfahren nach einem der Ansprüche 1, 11 oder 12, das umfasst:

    a. einen Schritt des Blockierens des Wachstums der Biomasse
    b. einen Schritt der Induzierung der Synthese der Phycobiliproteine
    c. einen Schritt der Präzipitation mithilfe von Salicylsäure
    d. einen Schritt der Solubilisierung des in Schritt c erhaltenen Präzipitats.

14. Verfahren nach einem der vorhergehenden Ansprüche, wobei das wässrige Extrakt von Phycobiliproteinen aus photosynthetisierenden Mikroalgen der Spezies *Arthospira Platensis* erhalten wird.

**Claims**

1. A process for obtaining a stable precipitate enriched in phycobiliproteins, comprising at least one precipitation step consisting of the addition of salicylic acid to an aqueous extract of phycobiliproteins, said precipitate having a phycobiliprotein content at least equal to 50% of the amount initially contained in said aqueous extract, and having a clarity at least equal to 70%.

2. The process according to claim 1, wherein said precipitate has a phycobiliprotein content at least equal to 50% of the amount initially contained in said aqueous extract and has a clarity at least equal to 80%, preferably 90%, or equal to 100%.

3. The process according to claim 1, wherein said precipitate has a phycobiliprotein content at least equal to 70% of the amount initially contained in said aqueous extract and is free of membrane fragments.

4. The process according to one of the preceding claims, wherein said aqueous extract of phycobiliproteins contains at least 1.5 g/l of said phycobiliproteins.

5. The process according to claim 1, wherein the salicylic acid is added to the aqueous extract of phycobiliproteins in a range of from 4 g/l to 20 g/l.

6. The process according to claim 1 or 3, wherein the salicylic acid is added to the aqueous extract of phycobiliproteins in a range of from 4 g/l to 13 g/l.

7. The process according to claim 1 or 2, wherein the salicylic acid is added to an aqueous extract of phycobiliproteins in a range of from 13 to 14 g/l.

8. The process according to one of the preceding claims, wherein a step of clarification by decanting prior to the step of precipitation by salicylic acid is added.

9. The process according to one of the preceding claims, wherein a step of dissolving the precipitate is added.

10. The process according to claim 9, wherein the precipitate is dissolved in a natural polyol chosen from glycerin, glycerol, or any derivative or any form of the polyol propane-1,2,3-triol (or 1,2,3-propanetriol).

11. The process according to claim 1, which comprises, beforehand, a step of enrichment in phycobiliproteins of a biomass of photosynthetic microalgae.

12. The process according to claim 11, wherein the prior step of enrichment in phycobiliproteins comprises an induction of the phycobiliprotein synthesis in a biomass of which the growth is blocked.

13. The process according to one of claims 1, 11 or 12, which comprises:

   a. a step of blocking the growth of the biomass
   b. a step of inducing the synthesis of the phycobiliproteins
   c. a step of precipitating using salicylic acid
   d. a step of dissolving the precipitate obtained in step c.

14. The process according to one of the preceding claims, wherein the aqueous extract of phycobiliproteins is obtained from *Arthrospira platensis* photosynthetic microalgae.

Figure 1

Figure 2

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5643585 A
- WO 2005065697 A
- WO 2014045177 A
- WO 2005065697 A1
- WO 09B6100 C
- WO 2014045177 A1

**Littérature non-brevet citée dans la description**

- **BRYANT D.A. ; GLAZER, A.N. ; EISERLING F.A.** Characterisation and Structural properties of the major biliproteins of Anabaena sp. *Arch. Microbiol,* 1976, vol. 110, 61-75

- **COSTA JAV ; COLLA LM ; DUARTE FILHO PF ; KABKE K ; WEBER A.** Modelling of Spirulina platensis growth in freshwater using response surface methodology. *World J Microbiol Biotechnol,* 2002, vol. 18, 603-607